# FASCICULE DE BREVET EUROPEEN

(11) **EP 0 611 326 B1**
(45) Date de publication et mention de la délivrance du brevet: **06.08.1997**
(21) Numéro de dépôt: 93909334.0
(22) Date de dépôt: 27.10.1992
(51) Int. Cl.: B01J 13/04, A61K 9/51

(54) **PROCEDE DE FABRICATION DE NANOCAPSULES A PAROI A BASE DE PROTEINES RETICULEES; NANOCAPSULES AINSI OBTENUES ET COMPOSITIONS COSMETIQUES, PHARMACEUTIQUES ET ALIMENTAIRES EN COMPORTANT APPLICATION**
HERSTELLUNGSVERFAHREN VON NANOKAPSELN MIT AUF VERNETZTEN PROTEINEN BASIERENDEN WÄNDEN; DERART HERGESTELLTE NANOKAPSELN UND DIESE ENTHALTENDE KOSMETIKA, PHARMAZEUTIKA UND LEBENSMITTEL
METHOD FOR PRODUCING NANOCAPSULES WITH CROSS-LINKED PROTEIN-BASED WALLS, NANOCAPSULES THEREBY OBTAINED, AND COSMETIC, PHARMACEUTICAL AND FOOD COMPOSITIONS USING SAME

(30) Priorité: 31.10.1991 FR 9113522
(43) Date de publication de la demande: 24.08.1994
(73) Titulaire: COLETICA, 69007 Lyon (FR)
(72) Inventeur: PERRIER, Eric, F-38200 Vienne (FR); HUC, Alain, F-69110 Ste-Foy-les-Lyon (FR)
(74) Mandataire: Portal, Gérard
(86) Numéro de dépôt international: FR9201003
(87) Numéro de publication internationale: WO9308908

(56) Documents cités:
- AU-A- 495 261
- FR-A- 2 642 329
- JOURNAL OF CONTROLLED RELEASE vol. 14, no. 2, 1 Octobre 1990, AMSTERDAM pages 111-131

## Description

La présente invention concerne essentiellement un procédé de fabrication de nanocapsules à paroi à base de protéine réticulée ainsi que les nanocapsules ainsi obtenues et des compositions cosmétiques, pharmaceutiques ou alimentaires en contenant.

On sait que l'encapsulation de substances actives est très importante en vue soit de protéger le principe actif, soit de permettre une libération lente ou différée du principe actif dans l'organisme.

On a proposé d'encapsuler les principes actifs dans des liposomes, les liposomes constituant une forme galénique enthousiasmante vue leur très bonne affinité avec les membranes cellulaires, leur très bonne biocompatibilité et leur taille submicronique.

Cependant, ces structures présentent de nombreuses limitations, voir même des inconvénients majeurs qui peuvent se résumer en les quatres points suivants :
- Un mauvais rendement d'encapsulation : les liposomes peuvent contenir ou véhiculer différents types de molécules : hydrophiles, lipophiles et amphiphiles. Cependant les rendements d'encapsulation sont très faibles dans tous les cas, ce qui, couplé au problème de la diffusion des principes actifs, diminue encore l'efficacité des liposomes et ne permet pas, dans bien des cas, d'envisager leur utilisation dans des applications thérapeutiques.
- Une mauvaise reproductibilité des préparations liposomales lorsque des productions industrielles sont à mettre en oeuvre.
- Une instabilité in vitro : elle peut se manifester de différentes façons : instabilité chimique des lipides, instabilité de la taille des liposomes, instabilité de leur structure, formation d'agrégats, relargage des actifs encapsulés, etc ...
- Une instabilité in vivo : l'influence des liquides biologiques sur les liposomes augmente très souvent les perméabilités membranaires de ceux-ci. Selon la voie d'administration utilisée, les liposomes peuvent être au contact de liquides biologiques aussi divers que le sang, les sucs digestifs, les liquides interstitiels... et doivent par conséquent être capables de résister à de nombreuses interactions. Or, le contact avec la plupart des liquides biologiques conduit à une augmentation nette de la perméabilité membranaire des liposomes. Par fusion imparfaite avec les cellules, ou par contact avec des sels, des enzymes, - lipases, phospholipases, acyl-transférases - des constituants plasmatiques, des sels biliaires, des sucs digestifs, ou par simples variations de pH, les liposomes peuvent relarguer de façon quasi instantanée leurs actifs dans le milieu environnant.

On a également proposé d'encapsuler les principes actifs dans des particules ou capsules de dimension de l'ordre de quelques microns. Par exemple, le déposant a proposé dans le document FR-A-2 642 329 la préparation de microcapsules à parois mixtes d'atélocollagènes et de glycosaminoglycannes pour l'encapsulation de principe actif. Cette méthode est tout-à-fait satisfaisante mais elle ne permet pas de préparer des capsules ayant une dimension submicronique, c'est-à-dire des capsules ayant une dimension nanométrique, dite nanoparticule.

Il a été proposé par ailleurs notamment par Couvreur et al dans Febs Letters (1977), 84, 323-326 des nanocapsules à paroi en polyacrylamide et par les mêmes auteurs dans J. Pharm. Pharmacol. (1979), 31, 331-332 des nanocapsules à paroi de polyméthyle et polyéthyle cyanoacrylate. De même, il a été proposé dans EP-A-0 274 961 de préparer des nanocapsules formant des systèmes colloïdaux à base d'un copolymère chlorure de vinyl et acétate de vinyl, de polyisobutylcyanoacrylate, de l'acide poly (d,l) lactique ; par polycondensation BEESTMAN et al ont proposé dans US-A-4 640 709 la préparation de sphères de petite taille dont les membranes sont constituées d'un matériel polymérique comme le polyurée, le polyamide, le polysulfonamide, le polyester, le polycarbonate et le polyuréthane.

Il est également connu par le document Journal of Controlled Release 14 (1990), 111-131, un procédé de fabrication de nanocapsules d'albumine comprenant la formation d'une nanoémulsion et la réticulation thermique de celle-ci à une temperature comprise entre 80-160°C, ainsi qu'une procédure modifiée comprenant une réticulation chimique à la 2,3 butanedione.

Il est encore connu par le document AU-84714/75 des nanoparticules comprenant une matrice réticulée de macromolécules d'origine naturelle, en particulier des protéines, ainsi que leur procédé de fabrication.

Cependant, la méthode de fabrication décrite prévoit la dissolution dans un solvent de la macromolécule qui est ensuite traitée avec un agent dissolvant dans des conditions de formation de particules et puis le traitement des particules avec un agent réticulant (hardening agent), voir pages 4 et 7. Les agents réticulants sont précisés en page 10, dernier paragraphe, et sont constitués par des aldéhydes qui conduisent à la formation de liaisons instables de type imines, tandis que dans le cas de la présente invention la réticulation a lieu avec un dichlorure d'acide ou un anhydride d'acide.

Cependant, bien qu'avec ces derniers documents on obtienne des capsules de taille nanométrique, un problème majeur réside dans le fait que ces particules ont généralement une mauvaise biocompatibilité, une mauvaise biodégradation in vitro et in vivo, pouvant conduire au stockage d'une forte concentration de particules dans certains organes, à une toxicité de certains monomères ou de certains sous-produits de polymérisation ou de certains sous-produits de dégradation et à une mauvaise protection des principes actifs lorsqu'ils ne sort qu'adsorbés à la surface des nanoparticules donnant ainsi un effet retard insuffisant.

Ainsi, la présente invention a pour but de résoudre le nouveau problème technique consistant en la fourniture d'une solution permettant la fabrication de particules de dimension nanométrique, dites nanoparticules, notamment sous forme de nanocapsules ou de nanosphères présentant une bonne biocompatibilité, une bonne biodégradation in vivo, une absence de toxicité ou une très faible toxicité, ainsi qu'une très bonne protection des principes actfs et un effet retard significatif.

La présente invention a encore pour but de résoudre le nouveau problème technique énoncé ci-dessus d'une manière simple, peu coûteuse, utilisable à l'échelle industrielle.

La présente invention permet pour la première fois de résoudre ces problèmes techniques d'une manière simple, peu coûteuse, fiable, utilisable à l'échelle industrielle et dans le domaine de la cosmétique, de la pharmacie, ou de l'agro-alimentaire, en obtenant des particules ou capsules de dimension submicronique, donc de taille inférieure à 1 µm, et notamment comprise entre 100 et 800 nanomètres environ.

Ainsi, selon un premier aspect, la présente invention fournit des particules ou capsules de dimension inférieure à un micromètre, dits nanoparticules ou nanocapsules, à paroi en protéines réticulées, caractérisées en ce qu'elles sont obtenues par une réaction de réticulation interfaciale entre une protéine et un agent réticulant comprenant au moins deux groupes acylants réagissant avec les groupes réactifs acylables de ladite protéine, on obtenant ainsi ladite nanoparticule ou nanocapsule à paroi à base de protéine réticulée.

D'autres caractéristiques de ces nanoparticules ou nanocapsules sont énoncées dans les sous-revendications qui sont incorporées ici en totalité par référence.

Selon un deuxième aspect, la présente invention fournit un procédé de fabrication de particules ou de capsules de dimension inférieure à in micromètre, dites nanoparticules ou nanocapsules, à paroi à base de protéines réticulées, caractérisée en ce qu'il comprend la réalisation d'une nanoémulsion comprenant une phase aqueuse contenant une protéine et une phase huileuse contenant un agent de réticulation interfaciale comportant au moins deux groupes acylants réagissant avec les groupes acylables de ladite protéine en formant ainsi des microparticules ou microcapsules dont la paroi est à base de protéines réticulées par ledit agent réticulant.

Selon une variante de réalisation, on ajoute un agent modificateur de viscosité de manière à diminuer la différence de viscosité entre les phases liquides en présence pour obtenir ladite nanoémulsion.

D'autres variantes résultent des sous-revendications de procédé et de la description suivante.

Selon une autre variante de réalisation, ce procédé est caractérisé en ce que l'agent modificateur de viscosité est capable de modifier la viscosité d'au moins 4 fois et de préférence d'au moins 10 fois, par rapport à la phase à laquelle ledit agent est ajouté.

Selon une autre variante, ce procédé est caractérisé en ce que, dans le cas de la formation d'une émulsion eau-dans-huile, ledit agent modificateur de viscosité est ajouté ou substitué à la phase huileuse de manière à augmenter la viscosité d'au moins 4 fois par rapport à la viscosité de la phase huileuse utilisée classiquement (FR-A-2 642 329).

Selon une autre variante, ce procédé est caractérisé en ce que dans le cas d'une émulsion huile-dans-eau, on diminue la viscosité de la phase aqueuse soit en diminuant la proportion en protéine, soit en ajoutant un agent modificateur de viscosité dans la phase aqueuse (agent fluidifiant de viscosité) de manière à diminuer sa viscosité et de préférence d'au moins 4 fois par rapport à la viscosité de la phase aqueuse utilisée classiquement (FR-A-2 642 329).

Selon une autre variante ce procédé est caractérisé en ce que l'on utilise comme protéine une protéine à effet filmogène, de préférence choisie parmi le groupe consistant d'une protéine animale telle que élastine, kératine, soie, albumine, protéines du lait, protéines de structure telles que collagène, notamment collagène sans télopeptide ou atélocollagène ou un glycosaminoglycanne ; une protéine végétale telle que protéine de blé, de maïs, d'avoine, d'amande ; et une protéine issue du milieu marin notamment extraite de poissons, d'algues ou encore du plancton ou microplancton.

Selon une autre variante ce procédé est caractérisé en ce que la protéine précitée a un poids moléculaire au moins égal à 50 000 Daltons, cette protéine étant utilisée seule ou en mélange.

Selon une autre variante, ce procédé est caractérisé en ce que la proportion de protéine dans la solution d'émulsion varie entre 0,1 et 5 % en poids par rapport au poids total de l'émulsion.

Selon une autre variante ce procédé est caractérisé en ce que la protéine est tout d'abord dissoute dans une solution aqueuse tamponée ayant un pH légèrement basique, de préférence compris entre environ 7,5 et environ 10,5.

Selon une autre variante ce procédé est caractérisé en ce que l'agent modificateur de viscosité précité est une huile visqueuse, en particulier choisie parmi l'huile de vaseline visqueuse, dont la viscosité est de préférence d'au moins 80 cp, de préférence encore d'au moins 200 cp ; ou un agent modificateur de viscosité des huiles telles que le stéarate de magnésium.

Selon une autre variante ce procédé est caractérisé en ce que, lors de l'étape d'émulsion, on utilise un agent tensioactif ou émulsionnant capable de former une nanoémulsion, de préférence du glycérol sorbitan hydroxyisostéarate.

Selon une autre variante ce procédé est caractérisé en ce que l'étape d'émulsion est réalisée sous une agitation à effet cisaillant, de préférence à au moins 20 000 tr/min, ou à effet de cavitation.

Selon une autre variante ce procédé est caractérisé en ce que l'on réalise une émulsion très fine en passant l'émulsion dans un homogénéiseur sous une pression d'au moins 400 bars, cet homogénéiseur étant de préférence une presse French.

Selon une autre variante ce procédé est caractérisé en ce que la protéine précitée comprend du collagène.

Selon une autre variante ce procédé est caractérisé en ce que la protéine précitée comprend de l'atélocollagène.

Selon une autre variante ce procédé est caractérisé en ce que la protéine précitée comprend un mélange d'atélocollagène et de glycosaminoglycanne.

Selon une autre variante ce procédé est caractérisé en ce que l'une des phases contient un principe actif cosmétique ou pharmaceutique, ou alimentaire, hydrosoluble, liposoluble, ou insoluble.

Selon encore une autre variante de réalisation, dans le cas de l'emploi d'une substance active hydrosoluble, on utilise un rapport d'émulsification huile/eau voisin de 6.

Selon une autre variante de réalisation, pour le cas où l'on utilise un principe actif liposoluble, on utilise une protéine à haut ou très haut poids moléculaire, c'est-à-dire d'au moins 50 000 Daltons, à une concentration telle que la viscosité de la solution obtenue soit faible, c'est-à-dire inférieure à 20 cp (mPa.s).

D'autre part, dans ce cas, on préfère utiliser un rapport d'émulsification eau/huile voisin de 20.

Par ailleurs, on emploie dans le cadre du procédé, tout agent réticulant bien connu de l'homme de l'art, tel que décrit notamment dans FR-2 642 329.

L'agent réticulant décrit dans FR-A-2 642 329 est un dichlorure d'acide ou un anhydride d'acide ou un acide carboxylique di- ou poly-basique.

Selon une caractéristique préférée, l'agent réticulant est choisi parmi le chlorure de téréphthaloyle, le chlorure d'acide phtalique, le chlorure d'acide sébacique, le chlorure d'acide succinique, le chlorure d'un acide tricarboxylique comme l'acide citrique, ou un anhydride d'acide comme l'anhydride succinique.

Selon un troisième aspect, la présente invention couvre également une composition cosmétique, pharmaceutique ou alimentaire, caractérisée en ce qu'elle comprend des nanocapsules à paroi à base de protéines réticulées telles que précédemment définies, de préférence obtenues par le procédé défini. De préférence, ces nanocapsules contiennent au moins en partie un principe actif, en particulier un principe actif cosmétique, pharmaceutique ou alimentaire, hydrosoluble, liposoluble ou insoluble.

D'autres buts, caractéristiques et avantages de l'invention apparaitront clairement à la lumière de la description explicative qui va suivre faite en référence à divers exemples de réalisation de l'invention donnés simplement à titre d'illustration et qui ne sauraient donc en aucune façon limiter la portée de l'invention. Dans les exemples, tous les pourcentages sont donnés en poids sauf indication contraire.

### Exemple 1 selon l'invention

### Préparation de nanocapsule à paroi en protéines à base d'un mélange atélocollagène/glycosaminoglycannes

### a) Fabrication du mélange nécessaire à la fabrication des nanocapsules

Ce mélange est préparé selon une procédure décrite dans FR-A-2 642 329, exemple 1 étapes a) à c).
- A partir de peaux de veaux fraîchement abattus, le collagène est extrait puis les télopeptides sont éliminés pour obtenir de l'atélocollagène.
- A partir de cloisons nasales de veaux, le chondroïtine 4 sulfate est extrait, dialysé puis lyophilisé.
- Les deux préparations précédentes sont avantageusement placées en tampon basique tel que carbonate ou phosphate ou tout autre substance permettant d'obtenir un pouvoir tampon entre 7,5 et 10,5. Puis les solutions sont mélangées de manière à obtenir par exemple, les concentrations finales suivantes :
   - Atélocollagène :: 1,6 %
   - Chondroïtine 4 sulfate :: 0,6 %
   - Carbonate de sodium anhydre :: 4,8 %
   - Parahydroxybenzoate de méthyle :: 0,4 %
   - Eau permutée :: qsp

Le pH de l'ensemble est porté entre 7,5 et 10,5, par exemple à 8,5, par ajout de HCl, 6N ou de NaOH,6N.

Un kilo de cette solution ainsi préparée est utilisé dans la suite de la fabrication.

### b) Préparation de l'agent réticulant

400 g de chlorure de téréphtaloyle sont broyés dans un mortier et sont rajoutés à 1 l de vaseline visqueuse CODEX. L'ensemble est agité par agitation mécanique.

### c) Emulsification

Dans une cuve inox réfrigérée, sont introduits 6 l d'huile de vaseline visqueuse CODEX d'indice de viscosité 250 cp (mPa.s) et de préférence 320 ml d'un tensioactif par exemple le glycérol sorbitan hydroxyisostéarate (Arlacel 780, ICI). L'ensemble est agité pendant quelques minutes.

La solution d'atélocollagène et de chondroïtine sulfate préparée est alors ajoutée et l'émulsification est réalisée en quelques minutes à 20 000 rpm à l'aide d'un Ultra-Turax^{R}.

### d) Réticulation

La solution contenant l'agent réticulant préparé à l'étape b est alors introduite dans l'émulsion. Les particules solides présentes dans celle-ci sont rajoutées également, et se dissoudront au cours du temps.

Après 5 min d'agitation à 20 000 rpm à l'Ultra-Turax^{R}, la solution est mise sous agitation mécanique à vitesse de rotation réduite, pendant 18 h au moins.

Les nanocapsules sont séparées par contrifugation en discontinu et le surnageant est éliminé (4000 rpm pendant 15 min).

### e) Lavages

Les nanocapsules sont lavées par cinq solutions successives d'une phase organique miscible avec l'huile de vaseline. Citons pour exemples le DRAGOXAT^{R} (DRAGOCO), le MYRISTATE D'ISOPROPYLE (STEARINIERIE DUBOIS), les triglycérides (STEARINERIE DUBOIS), etc.

Au cours de chaque lavage, 100 ml de nanocapsules sont rajoutés à 500 ml de phase organique. L'ensemble est agité pendant quelques minutes puis centrifugé (4000 rpm pendant 15 min).

Les nanocapsules obtenues peuvent être mises en suspension par exemple dans des gels de protéine ou de polysaccharide, ou dans une phase huileuse.

### Exemple 2 de l'invention

### Préparation de nanocapsules contenant un principe actif hydrosoluble ou insoluble

On procède comme décrit à l'exemple 1 si ce n'est qu'à la solution fabriquée en a) on peut rajouter de nombreux principes actifs, comme par exemple :
- Ex 2A :: 64 g de GLYCENTANE^{R} (Bioética)
- Ex 2B :: 64 g de GINKGO BILOBA (Alban Muller Int.)
- Ex 2C :: 32 g de GLUCOSE (MERCK)
- Ex 2D :: 32 g d'un acide aminé tel que la L-Glutamine
- Ex 2E :: 32 g de CAFEINE (SIGMA).

### Exemple 3 de l'invention

### Préparation de nanocapsules à paroi en protéines à base d'atélocollagène

On procède comme décrit à l'exemple 1. Cependant, on utilise comme seule protéine macromoléculaire, l'atélocollagène à une concentration de 2 %.

### Exemple 4 de l'invention

### Nanocapsules à l'élastine

On procède comme décrit à l'exemple 1 si ce n'est que l'on utilise comme protéine de l'élastine.

On peut également choisir une protéine parmi les protéines animales telles qu'élastine, kératine, soie, albumine, protéines du lait, les protéines végétales telles que protéines de blé, de maïs, d'avoine, d'amande ou les protéines issus du milieu marin telles que collagène ou autres protéines extraits de poissons, protéines d'algues, microplancton.

### Exemple 5 de l'invention

Tous les exemples décrits précédemment peuvent être modifiés en utilisant d'autres méthodes d'émulsification.

Ainsi, dans l'exemple 1 en particulier, l'étape d'émulsification c) est modifiée et après un léger brassage par agitation mécanique, l'ensemble est passé une ou plusieurs fois dans un homogénéisateur haute pression.

Les pressions utilisées peuvent se situer entre 400 et 1000 bars mais se situent préférentiellement autour de 700 bars. Les homogénéisateurs simple et double effets peuvent être utilisés indifféremment mais les simples seront préférés pour des protéines très filmogènes.

Les exemples d'homogénéisateurs haute pression qui ont été utilisés sont le Lab 60 (APV), le SHL 05 (ALPHA-LAVAL), ou le SODEXIM 2720 ou 2735 (SODEXIM). Après un traitément d'homogénéisation à 700 bars par exemple, on obtient des nanocapsules ayant une dimension inférieure à 1 µm, comprise entre 200 et 800 nanomètres.

De même, d'autres appareils utilisant d'autres principes, peuvent également permettre l'obtention de forces de cavitation élevées. A l'aide de celles-ci, il est alors possible d'obtenir des nanoémulsions (exemples : SONICATEUR^{R} BRANDSON, SONOLATOR^{R} de SONIC Corp.).

### Exemple 6 de l'invention

Dans l'étape d'émulsification de l'exemple 1, on utilise un agent viscosant des huiles pour augmenter la viscosité de la solution d'émulsification par exemple le stéarate de magnésium à une proportion de 2 % en poids. On obtient des nanocapsules de dimension comprise entre 200 et 800 nanomètres.

### Exemple 7

### Préparation de nanocapsules contenant des actifs liposolubles

a) A 250 ml de la solution décrite dans l'exemple 1 en a), on rajoute 750 ml d'eau déminéralisée.
b) On rajoute à 25 ml d'huile de bourrache, 5 ml de dichlorure de sébacoyle et on mélange l'ensemble par agitation mécanique.
c) Les solutions a) et b) sont additionnées en continu et envoyées dans un homogénéisateur haute pression du type Lab 60 (APV). Les pressions d'homogénéisation utilisées sont comprises entre 300 et 1000 bars, par exemple 800 bars, et plusieurs homogénéisations successives ont été effectuées, avec des valves simple et double effets. Les sphères obtenues sont de taille inférieure à un micron, sont remarquablement stables et vu leur faible taille ne décantent pas dans un milieu dilué de stockage.

### Exemple 8

### Autres actifs liposolubles

Dans l'exemple 7, les 25 ml d'huile de bourrache peuvent être substitués par :
- Ex 8a :: 25 ml myristate d'éthyle
- Ex 8b :: 25 ml myristate d'isopropyle
- Ex 8c :: 25 ml d'huile de vaseline fluide
- Ex 8d :: 25 ml d'oléate d'éthyle
- Ex 8e :: 25 ml d'acétate de vitamine E
- Ex 8f :: 25 ml de benzoate de benzyle.

### Exemple 9

### Test d'objectivation

Relargage in vivo d'une substance encapsulée dans des nanocapsules selon l'invention par rapport à des microcapsules.

Les résultats des tests d'objectivation les plus convaincants fournis par les nanocapsules sont incontestablement ceux liés à une modification de la distribution spatio-temporelle de la substance active. Cette modification peut être liée à la taille des particules qui sont alors spécifiquement véhiculées dans certaines parties de l'organisme (système réticulo-endothélial, tissus hépatiques), mais elle peut être également liée au rôle de réservoir à actifs que peuvent jouer les nanocapsules Dans ce dernier cas, un relargage temporisé de l'actif peut permettre d'atteindre une forte biodisponibilité du principe actif, une assimilation plus intense, ainsi qu'une élimination beaucoup plus progressive des déchets issus de la métabolisation du principe actif.

L'étude comparative décrite ci-dessous a permis aux inventeurs d'évaluer la présence et l'intensité de l'effet retard, obtenu avec des capsules de taille micrométrique et avec des capsules de taille nanométrique selon l'invention.

### a) Matériels et méthodes

La peau dorsale de rats (WISTAR mâles, environ 300 g), a été traitée à l'aide d'une émulsion eau dans huile, la phase huileuse étant de l'huile de vaseline visqueuse codex d'indice de viscosité d'environ 250 cp (mPa.s) (TISCCO) et la phase aqueuse étant représentée par l'une ou l'autre des solutions ci-dessous :
- un mélange collagène/glycosaminoglycanne (en abrégé Coll/GAG) renfermant de l'acide para-aminobenzoïque radioactif (PABA*)
- des microcapsules de type A renfermant du PABA* (taille moyenne 50 µm) telles que préparées selon la méthode décrite à l'exemple 1 du brevet FR-A-2 642 329
- des nanocapsules renfermant du PABA* (taille comprise entre 100 et 800 nm) préparées selon la méthode de l'exemple 1 ci-dessus.

Après application sur une surface constante, du produit a tester, la libération de l'acide a été suivie par mesure de la radioactivité contenue dans l'urine récoltée quotidiennement pour chaque animal.

Pour chacune des trois solutions décrites ci-dessus, deux rats ont été traités par 0,3 g d'émulsion, possédant une radioactivité spécifique voisine de 3.10⁶ cpm/g dans le cas de la solution collagène/GAG et des microcapsules, et voisine de 6.10⁵ cpm/g dans le cas des nanocapsules de l'invention.

### b) Résultats

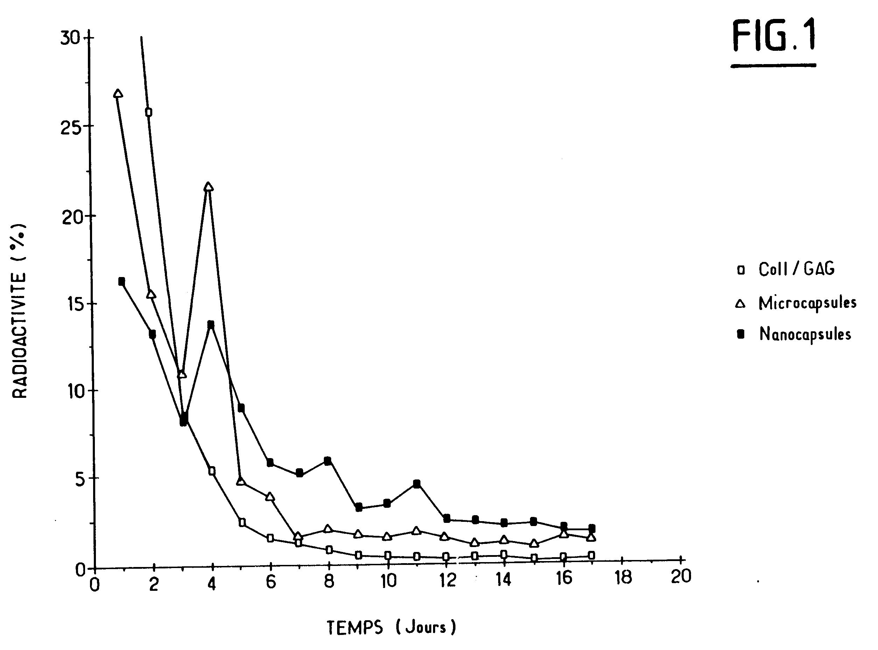

Après application du composé radioactif, encapsulé ou non encapsulé, la radioactivité a été mesurée chaque jour dans l'urine des rats. Les courbes montrant l'évolution de cette radioactivité en fonction du temps (en jours) sont représentées sur la figure 1.

Les valeurs rapportées sur ce graphique représentent la radioactivité mesurée dans les urines, divisée par la radioactivité totale récupérée dans les urines et dans la peau. Cette radioactivité totale récupérée est comptabilisée après les 17 jours de mesures et après sacrifice des animaux.

Ce graphique décrit l'effet retard observé avec les microcapsules et les nanocapsules sur le ralargage du PABA*. Le fort relargage des premiers jours, constaté avec la solution de collagène/GAG est moins intense avec les microcapsules et très faible avec les nanocapsules. Le PABA* s'élimine beaucoup plus lentement lorsqu'il est encapsulé dans les microcapsules et encore moins rapidement lorsqu'il est encapsulé dans les nanocapsules.

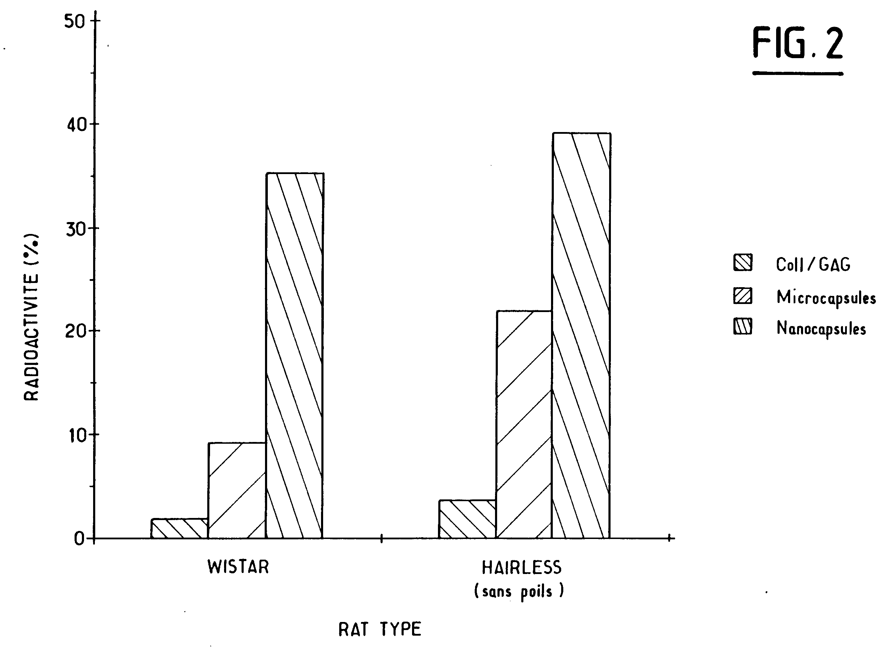

17 jours après le traitement, les rats sont sacrifiés, la peau ayant reçue l'application est hydrolysée, puis la radioactivité est mesurée. Les résultats obtenus ont été rassemblés dans la figure 2 et montrent que la radioactivité contenue dans les tissus cutanés est faible (voisine de 4 %) lorsque le PABA* n'a pas été encapsulé, beaucoup plus forte (voisin de 10 %) lorsque des microcapsules ont été utilisées, et encore plus intense (> 20 %) lorsque des nanocapsules ont encapsulé la substance radioactive.

Cette différence très nette a été confirmée dans une autre expérience qui a été menée où des rats nus mâles de 300 g ont été utilisés dans le même type d'expérimentation.

### c) Conclusions

- La vitesse d'élimination de la radioactivité retrouvée dans les urines étant directement reliée à la vitesse de l'absorption cutanée, tout retard dans l'élimination peut être considéré comme étant dû à l'effet retard des sphères encapsulant l'élément radioactif. Cet effet retard constaté in vivo sur le rat est très net avec des microcapsules de 50 µm mais est encore plus intense avec des nanocapsules (dont la taille varie entre 800 nm et 100 nm).
- La radioactivité mesurée au niveau du tissu cutané, après application d'un élément radioactif, encapsulé ou non, est une mesure de la biodisponibilité de cet élément, c'est-à-dire de sa capacité à être intégré dans le métabolisme cutané. Après application d'une quantité trop forte de PABA*, l'élimination est très rapide, les tissus cutanés ne pouvant accepter qu'une fraction aliquote du produit appliqué.

Lorsque des microcapsules ou des nanocapsules sont utilisées, il y a relargage temporisé de PABA* qui peut alors être métabolisé en de plus grandes quantités dans les tissus cutanés.

C'est ce que l'on observe avec les nanocapsules de l'invention et dans une moindre mesure avec les microcapsules.

Si la taille des capsules est suffisamment petite pour qu'il n'y ait pas éclatement lors de l'application (diamètre inférieur à 100 µm), il est donc possible d'obtenir grâce aux nanocapsules une amélioration de la biodisponibilité des actifs cosmétiques.

La présente invention couvre tous les moyens constituant des équivalents techniques des moyens décrits. En particulier, dans la description et les revendications, le mot "nanocapsule" n'est pas limité à des capsules proprement dites mais couvre des sphères ou des particules dont la dimension est nanométrique.

## Revendications

1. Particule ou capsule de dimension inférieure à 1 µm, dite nanoparticule ou nanocapsule, à paroi en protéine réticulée, caractérisée en ce qu'elle est obtenue par une réaction de réticulation interfaciale entre une protéine et un agent réticulant comprenant au moins deux groupes acylants réagissant avec les groupes réactifs acylables de ladite protéine, en obtenant ainsi ladite nanoparticule ou nanocapsule à paroi à base de protéine réticulée.

2. Nanoparticule ou nanocapsule selon la revendication 1, caractérisée en ce que la protéine précitée a un poids moléculaire au moins égal à 50 000 Daltons, cette protéine étant utilisée seule ou en mélange.

3. Nanoparticule ou nanocapsule selon la revendication 1 ou 2, caractérisée en ce que la protéine précitée est choisie parmi le groupe consistant d'une protéine animale telle qu'élastine, kératine, soie, albumine, protéine du lait, protéine de structure telle que collagène, notamment collagène sans télopeptide ou atélocollagène; une protéine végétale telle que protéine de blé, de maïs, d'avoine, d'amande; et une protéine issue du milieu marin notamment de poisson ou d'algue ou encore du plancton ou micro-plancton.

4. Nanoparticule ou nanocapsule selon les revendications 1 à 3, caractérisée en ce que la protéine précitée comprend du collagène, de l'atélocollagène ou un mélange d'atélocollagène et de glycosaminoglycanne.

5. Nanoparticule ou nanocapsule selon l'une des revendications précédentes, caractérisée en ce qu'elle contient un principe actif cosmétique, pharmaceutique ou alimentaire, hydrosoluble, liposoluble ou insoluble.

6. Nanoparticule ou nanocapsule selon la revendication 4 ou 5, caractérisée en ce que le glycosaminoglycanne précité est le chondroïtine-4-sulfate.

7. Nanoparticule ou nanocapsule selon l'une des revendications 1 à 6, caractérisée en ce que l'agent réticulant comprenant au moins deux groupes acylants capables de réagir avec les groupes acylables de la protéine est choisi parmi le groupe consistant d'un dichlorure d'acide, en particulier le chlorure de terephthaloyle, le chlorure d'acide phtalique, le chlorure d'acide sébacique, le chlorure d'acide succinique, le chlorure d'un acide tricarboxylique comme l'acide citrique, ou un anhydride d'acide tel que l'anhydride succinique.

8. Composition cosmétique, pharmaceutique ou alimentaire, caractérisée en ce qu'elle comprend une nanoparticule ou une nanocapsule telle que définie dans l'une quelconque des revendications précédentes.

9. Procédé de fabrication de particule ou de capsule de dimension inférieure à 1 µm dite nanoparticule ou nanocapsule, à paroi à base de protéine réticulée, caractérisé en ce qu'il comprend la réalisation d'une nanoémulsion comprenant une phase aqueuse contenant une protéine et une phase huileuse contenant un agent de réticulation interfaciale comportant au moins deux groupes acylants réagissant avec les groupes acylables de ladite protéine en formant ainsi des microparticules ou microcapsules dont la paroi est à base de protéine réticulée par ledit agent réticulant.

10. Procédé selon la revendication 9, caractérisé en ce qu'on ajoute un agent modificateur de viscosité dans l'une des deux phases de manière à diminuer la différence de viscosité entre les phases liquides en présence pour obtenir ladite nanoémulsion.

11. Procédé selon la revendication 8 ou 9, caractérisé en ce qu'on réalise la nanoémulsion en passant une émulsion de la phase aqueuse et de la phase huileuse dans un homogénéiseur sous une pression d'au moins 400 bars.

12. Procédé selon l'une des revendications 9 à 11, caractérisé en ce qu'on réalise la nanoémulsion sous une agitation à effet cisaillant, de préférence au moins 20.000 tour/min, ou à effet de cavitation.

13. Procédé selon l'une des revendications 9 à 11, caractérisée en ce que la protéine précitée a un poids moléculaire au moins égal à 50 000 Daltons, cette protéine étant utilisée seule ou en mélange.

14. Procédé selon l'une des revendications 9 à 11, caractérisé en ce que l'agent modificateur de viscosité est capable de modifier la viscosité d'au moins 4 fois et de préférence d'au moins 10 fois, par rapport à la phase à laquelle ledit agent est ajouté.

15. Procédé selon l'une des revendications 9 à 14, caractérisé en ce que dans le cas de la formation d'une émulsion eau-dans-huile, ledit agent modificateur de viscosité est ajouté à ou constitue la phase huileuse de manière à augmenter la viscosité d'au moins 4 fois par rapport à la viscosité de la phase huileuse utilisée classiquement.

16. Procédé selon l'une des revendications 9 à 14, caractérisé en ce que dans le cas d'une émulsion huile-dans-eau, on diminue la viscosité de la phase aqueuse soit en diminuant la proportion en protéine, soit par l'ajout d'un agent modificateur de viscosité de manière à diminuer sa viscosité, de préférence d'au moins 4 fois, par rapport à la viscosité de la phase aqueuse utilisée habituellement.

17. Procédé selon l'une des revendications 9 à 16, caractérisé en ce qu'on utilise comme protéine une protéine à effet filmogène, de préférence choisie parmi le groupe consistant d'une protéine animale telle qu'élastine, kératine, soie, albumine, protéines du lait, protéines de structure telle que collagène, notamment collagène sans télopeptide ou atélocollagène ou un glucosaminoglycanne ; une protéine végétale telle que protéine de blé, maïs, d'avoine d'amande ; et une protéine issue du milieu marin notamment de poissons ou d'algues ou encore du plancton ou microplancton.

18. Procédé selon l'une des revendications 9 à 17, caractérisé en ce que la proportion de protéine dans la solution d'émulsion varie entre 0,1 et 5% en poids par rapport au poids total de l'émulsion.

19. Procédé selon l'une des revendications 9 à 18, caractérisé en ce que l'agent modificateur de viscosité précitée est une huile visqueuse, en particulier choisie parmi l'huile de vaseline visqueuse, dont la viscosité est de préférence d'au moins 80 cp, de préférence encore d'au moins 200 cp ; ou un agent modificateur de viscosité des huiles tel que le stéarate de magnésium.

20. Procédé selon l'une des revendications 9 à 19, caractérisé en ce que, lors de l'étape d'émulsion, on utilise un agent tensioactif ou émulsionnant capable de former une nanoémulsion, de préférence du glycérol sorbitan hydroxyisostéarate.

21. Procédé selon l'une des revendications 9 à 20, caractérisé en ce que la protéine précitée comprend du collagène, de l'atélocollagène, un mélange d'atélocollagène et de glycosaminoglycanne.

22. Procédé selon l'une des revendications 9 à 21, caractérisé en ce que l'une des phases contient un principe actif cosmétique, pharmaceutique ou alimentaire, hydrosoluble, liposoluble ou insoluble.

23. Procédé selon l'une des revendications 9 à 22, caractérisé en ce que lorsque le principe actif est hydrosoluble, on utilise un rapport d'émulsion huile/eau voisin de 6.

24. Procédé selon l'une des revendications 9 à 23, caractérisé en ce que lorsque le principe actif est liposoluble, on utilise un rapport d'émulsion eau/huile voisin de 20.

25. Procédé selon l'une des revendications 9 à 24, caractérisé en ce que, lorsque le principe actif est liposoluble, on utilise une protéine à haut poids moléculaire au moins égal à 50 000 Daltons, la concentration de la protéine est telle que la viscosité de la solution aqueuse obtenue soit inférieure à 20 cp (mPa.s).

26. Procédé selon l'une des revendications 9 à 25 caractérisé en ce que l'agent réticulant est tel que défini à la revendication 7.

## Claims

1. A particle or capsule of dimensions of less than 1 µm, called nanoparticle or nanocapsule, with a crosslinked protein-based wall, characterized in that it is obtained by an interfacial crosslinking reaction between a protein and a crosslinking agent comprising at least two acylating groups that react with the reactive acylatable groups of said protein, thus giving said nanoparticle or nanocapsule with a crosslinked protein-based wall.

2. A nanoparticle or nanocapsule according to claim 1, characterized in that the abovementioned protein has a molecular weight of at least 50,000 Daltons, this protein being used by itself or in admixture.

3. The nanoparticle or nanocapsule according to claim 1 or 2, characterized in that the abovementioned protein is selected from the group consisting of an animal protein such as elastin, keratin, silk, albumin, milk protein or structural protein such as collagen, especially collagen without telopeptide or atelocollagen; a vegetable protein such as wheat, maize, oat or almond protein; and a protein originating from the marine environment, especially from fish or algae or else from plankton or microplankton.

4. The nanoparticle or nanocapsule according to claims 1 to 3, characterized in that the abovementioned protein comprises collagen, atelocollagen or a mixture of atelocollagen and glycosaminoglycan.

5. The nanoparticle or nanocapsule according to one of the preceding claims, characterized in that it contains a water-soluble, liposoluble or insoluble active principle for use in cosmetics, pharmaceuticals or foodstuffs.

6. The nanoparticle or nanocapsule according to claim 4 or 5, characterized in that the abovementioned glycosaminoglycan is chondroitine 4-sulfate.

7. The nanoparticle or nanocapsule according to one of claims 1 to 6, characterized in that the crosslinking agent comprising at least two acylating groups capable of reacting with the acylatable groups of the protein is selected from the group consisting of an acid dichloride, in particular terephthaloyl chloride, phthalic acid chloride, sebacic acid chloride, succinic acid chloride, the chloride of a tricarboxylic acid such as citric acid, or an acid anhydride such as succinic anhydride.

8. A cosmetic, pharmaceutical or food composition, characterized in that it comprises a nanoparticle or a nanocapsule as defined in any one of the preceding claims.

9. A process for the production of particles or of capsules of dimensions of less than 1 µm, called nanoparticles or nanocapsules, with crosslinked protein-based walls, characterized in that it comprises preparing a nanoemulsion comprising an aqueous phase containing a protein and an oily phase containing an interfacial crosslinking agent comprising at least two acylating groups that react with the acylatable groups of said protein, thus forming microparticles or microcapsules whose walls are based on a protein crosslinked by said crosslinking agent.

10. The process according to claim 9, characterized in that a viscosity modifier is added to one of the two phases so as to reduce the viscosity difference between the liquid phases present in order to obtain said nanoemulsion.

11. The process according to claim 8 or 9, characterized in that nanoemulsion is produced by passing an emulsion of the aqueous phase and of the oily phase through a homogenizer under a pressure of at least 400 bar.

12. The process according to one of claims 9 to 11, characterized in that nanoemulsion is carried out by means of stirring with a shear effect, preferably at least 20,000 rpm, or with a cavitation effect.

13. The process according to one of claims 9 to 11, characterized in that the abovementioned protein has a molecular weight of at least 50,000 Daltons, this protein being used by itself or in admixture.

14. The process according to one of claims 9 to 11, characterized in that the viscosity modifier is capable of modifying the viscosity by a factor of at least 4 and preferably at least 10, relative to the phase to which said modifier is added.

15. The process according to one of claims 9 to 14, characterized in that, in the case of the formation of a water-in-oil emulsion, said viscosity modifier is added to or substituted for the oily phase so as to increase the viscosity by a factor of at least 4, relative to the viscosity of the oily phase conventionally used.

16. The process according to one of claims 9 to 14, characterized in that, in the case of an oil-in-water emulsion, the viscosity of the aqueous phase is reduced either by reducing the proportion of protein or by adding a viscosity modifier so as to reduce its viscosity, preferably by a factor of at least 4, relative to the viscosity of the aqueous phase conventionally used.

17. The process according to one of claims 9 to 16, characterized in that the protein used is a protein with a film-forming effect which is preferably selected from the group consisting of animal protein such as elastin, keratin, silk, albumin, milk proteins or structural proteins such as collagen, especially collagen without telopeptide, atelocollagen, or a glycosaminoglycan; a vegetable protein such as wheat, maize, oat or almond protein; and a protein originating from the marine environment, especially from fish or algae or else from plankton or microplankton.

18. The process according to one of claims 9 to 17, characterized in that the proportion of protein in the emulsion solution varies between 0.1 and 5% by weight, based on the total weight of the emulsion.

19. The process according to one of claims 9 to 18, characterized in that the abovementioned viscosity modifier is a viscous oil selected in particular from viscous liquid paraffin whose viscosity is preferably at least 80 cp and particularly preferably at least 200 cp; or a viscosity modifier for oils, such as magnesium stearate.

20. The process according to one of claims 9 to 19, characterized in that a surfactant or emulsifying agent capable of forming a nanoemulsion, preferably glycerol sorbitan hydroxyisostearate, is used in the emulsion step.

21. The process according to one of claims 9 to 20, characterized in that the abovementioned protein comprises collagen, atelocollagen, a mixture of atelocollagen and glycosaminoglycan.

22. The process according to one of claims 9 to 21, characterized in that one of the phases contains a water-soluble, liposoluble or insoluble active principle for use in cosmetics, pharmaceuticals or foodstuffs.

23. The process according to one of claims 9 to 22, characterized in that, when the active principle is water-soluble, an oil/water emulsion ratio close to 6 is used.

24. The process according to one of claims 9 to 23, characterized in that, when the active principle is liposoluble, a water/oil emulsion ratio close to 20 is used.

25. The process according to one of claims 9 to 24, characterized in that, when the active principle is liposoluble, a protein with a high molecular weight of at least 50,000 Daltons is used, the concentration of the protein is such that the viscosity of the aqueous solution obtained is less than 20 cp (mPa.s).

26. The process according to one of claims 9 to 25, characterized in that the crosslinking agent is as defined in claim 7.

## Patentansprüche

1. Teilchen oder Kapsel mit einer Abmessung von weniger als 1 µm, welches bzw. welche als Nanoteilchen oder Nanokapsel bezeichnet wird, mit einer Wand aus vernetztem Protein, dadurch gekennzeichnet, daß es bzw. sie durch eine Grenzflächen-Vernetzungsreaktion zwischen einem Protein und einem Vernetzungsmittel erhalten wird, das zumindest zwei Acylierungsgruppen umfaßt, die mit den acylierbaren reaktiven Gruppen des Proteins reagieren, wobei so das Nanoteilchen oder die Nanokapsel mit der Wand auf der Basis von vernetztem Protein erhalten wird.

2. Nanoteilchen oder Nanokapsel nach Anspruch 1, dadurch gekennzeichnet, daß das Protein eine Molmasse von zumindest 50 000 Dalton aufweist, wobei dieses Protein allein oder in Mischung verwendet wird.

3. Nanoteilchen oder Nanokapsel nach Anspruch 1 oder 2, dadurch gekennzeichnet, daß das Protein ausgewählt ist aus der Gruppe bestehend aus tierischem Protein, wie Elastin, Keratin, Seide, Albumin, Milchprotein, Protein mit einer Struktur wie Kollagen, insbesondere Kollagen ohne Telopeptid oder Atelokollagen; pflanzlichem Protein, wie Weizen-, Mais-, Hafer-, Mandelprotein; und Protein, das aus einer marinen Umgebung stammt, insbesondere von Fischen oder Algen oder auch Plankton oder Mikroplankton.

4. Nanoteilchen oder Nanokapsel nach einem der Ansprüche 1 bis 3, dadurch gekennzeichnet, daß das Protein Kollagen, Atelokollagen oder eine Mischung von Atelokollagen und Glykosaminoglykan umfaßt.

5. Nanoteilchen oder Nanokapsel nach einem der vorhergehenden Ansprüche, dadurch gekennzeichnet, daß es bzw. sie einen kosmetischen, pharmazeutischen oder alimentären, wasserlöslichen, fettlöslichen oder unlöslichen aktiven Bestandteil enthält.

6. Nanoteilchen oder Nanokapsel nach Anspruch 4 oder 5, dadurch gekennzeichnet, daß das Glykosaminoglykan Chondroitin-4-sulfat ist.

7. Nanoteilchen oder Nanokapsel nach einem der Ansprüche 1 bis 6, dadurch gekennzeichnet, daß das Vernetzungsmittel, das zumindest zwei Acylierungsgruppen umfaßt, die mit den acylierbaren Gruppen des Proteins reagieren können, ausgewählt ist aus der Gruppe bestehend aus einem Säuredichlorid, insbesondere Terephthaloylchlorid, Phthalsäurechlorid, Sebacinsäurechlorid, Bernsteinsäurechlorid, einem Chlorid einer Tricarbonsäure, wie Citronensäure, oder einem Säureanhydrid, wie Bernsteinsäureanhydrid.

8. Kosmetische, pharmazeutische oder alimentäre Zusammensetzung, dadurch gekennzeichnet, daß sie ein Nanoteilchen oder eine Nanokapsel, wie in einem der vorhergehenden Ansprüche definiert, umfaßt.

9. Verfahren zur Herstellung eines Teilchens oder einer Kapsel mit einer Abmessung von weniger als 1 µm, das bzw. die als Nanoteilchen oder Nanokapsel bezeichnet wird, mit einer Wand auf der Basis von vernetztem Protein, dadurch gekennzeichnet, daß es die Erzeugung einer Nanoemulsion umfaßt, umfassend eine wässerige Phase, die ein Protein enthält, und eine Ölphase, die ein Grenzflächen-Vernetzungsmittel enthält, das zumindest zwei Acylierungsgruppen umfaßt, die mit den acylierbaren Gruppen des Proteins reagieren, wobei so Mikroteilchen oder Mikrokapseln gebildet werden, deren Wand auf durch das Vernetzungsmittel vernetztem Protein basiert.

10. Verfahren nach Anspruch 9, dadurch gekennzeichnet, daß ein Viskositätsmodifikator einer der beiden Phasen zugesetzt wird, um die Viskositätsdifferenz zwischen den vorliegenden flüssigen Phasen zu verringern, wobei die Nanoemulsion erhalten wird.

11. Verfahren nach Anspruch 8 oder 9, dadurch gekennzeichnet, daß die Nanoemulsion erzeugt wird, indem eine Emulsion der wässerigen Phase und der Ölphase in einen Homogenisator unter einem Druck von zumindest 400 bar geführt wird.

12. Verfahren nach einem der Ansprüche 9 bis 11, dadurch gekennzeichnet, daß die Nanoemulsion unter Rühren mit einer Scherwirkung, vorzugsweise bei zumindest 20 000 UpM, oder mit einer Kavitationswirkung erzeugt wird.

13. Verfahren nach einem der Ansprüche 9 bis 11, dadurch gekennzeichnet, daß das Protein eine Molmasse von zumindest 50 000 Dalton aufweist, wobei dieses Protein allein oder in Mischung verwendet wird.

14. Verfahren nach einem der Ansprüche 9 bis 11, dadurch gekennzeichnet, daß der Viskositätsmodifikator die Viskosität zumindest 4-fach und vorzugsweise 10-fach in bezug auf die Phase modifizieren kann, der er zugesetzt wird.

15. Verfahren nach einem der Ansprüche 9 bis 14, dadurch gekennzeichnet, daß im Fall der Bildung einer Wasser-in-Öl-Emulsion der Viskositätsmodifikator der Ölphase zugesetzt wird oder diese bildet, um die Viskosität zumindest 4-fach in bezug auf die Viskosität der klassisch verwendeten Ölphase zu erhöhen.

16. Verfahren nach eine der Ansprüche 9 bis 14, dadurch gekennzeichnet, daß im Fall einer Öl-in-Wasser-Emulsion die Viskosität der wässerigen Phase entweder durch die Verminderung der Protein-Menge oder durch den Zusatz eines Viskositätsmodifikators zur Verringerung ihrer Viskosität, vorzugsweise zumindest um das 4-fache, in bezug auf die Viskosität der üblicherweise verwendeten wässerigen Phase verringert wird.

17. Verfahren nach einem der Ansprüche 9 bis 16, dadurch gekennzeichnet, daß als Protein ein Protein mit einer Filmbildnerwirkung, vorzugsweise ausgewählt aus der Gruppe bestehend aus tierischem Protein, wie Elastin, Keratin, Seide, Albumin, Milchproteinen, Proteinen mit einer Struktur wie Kollagen, insbesondere Kollagen ohne Telopeptid oder Atelokollagen oder Glykosaminoglykan; pflanzlichem Protein, wie Weizen-, Mais-, Hafer-, Mandelprotein; und Protein, das aus einer marinen Umgebung stammt, insbesondere von Fischen oder Algen oder auch Plankton oder Mikroplankton, verwendet wird.

18. Verfahren nach einem der Ansprüche 9 bis 17, dadurch gekennzeichnet, daß die Menge des Proteins in der Emulsionslösung zwischen 0,1 und 5 Masse-%, bezogen auf die Gesamtmasse der Emulsion, variiert.

19. Verfahren nach einem der Ansprüche 9 bis 18, dadurch gekennzeichnet, daß der Viskositätsmodifikator ein viskoses Öl, insbesondere ausgewählt aus viskosem Vaselineöl, dessen Viskosität vorzugsweise zumindest 80 cp, bevorzugter zumindest 200 cp, beträgt, oder ein Viskositätsmodifikator von Ölen, wie Magnesiumstearat, ist.

20. Verfahren nach einem der Ansprüche 9 bis 19, dadurch gekennzeichnet, daß im Emulsionsschritt ein grenzflächenaktiver Stoff oder ein Emulgator verwendet wird, der eine Nanoemulsion bilden kann, vorzugsweise Glycerinsorbitanhydroxyisostearat.

21. Verfahren nach einem der Ansprüche 9 bis 20, dadurch gekennzeichnet, daß das Protein Kollagen, Atelokollagen, eine Mischung von Atelokollagen und Glykosaminoglykan umfaßt.

22. Verfahren nach einem der Ansprüche 9 bis 21, dadurch gekennzeichnet, daß eine der Phasen einen kosmetischen, pharmazeutischen oder alimentären, wasserlöslichen, fettlöslichen oder unlöslichen aktiven Bestandteil enthält.

23. Verfahren nach einem der Ansprüche 9 bis 22, dadurch gekennzeichnet, daß, wenn der aktive Bestandteil wasserlöslich ist, ein Öl/Wasser-Emulsionsverhältnis von etwa 6 verwendet wird.

24. Verfahren nach einem der Ansprüche 9 bis 23, dadurch gekennzeichnet, daß, wenn der aktive Bestandteil fettlöslich ist, ein Wasser/Öl-Emulsionsverhältnis von etwa 20 verwendet wird.

25. Verfahren nach einem der Ansprüche 9 bis 24, dadurch gekennzeichnet, daß, wenn der aktive Bestandteil fettlöslich ist, ein Protein mit hoher Molmasse von zumindest 50 000 Dalton verwendet wird, wobei die Protein-Konzentration derart ist, daß die Viskosität der erhaltenen wässerigen Lösung weniger als 20 cp (mPa.s) beträgt.

26. Verfahren nach einem der Ansprüche 9 bis 25, dadurch gekennzeichnet, daß das Vernetzungsmittel wie in Anspruch 7 definiert ist.
